# FASCICULE DE BREVET EUROPEEN

(11) **EP 2 811 938 B1**
(45) Date de publication et mention de la délivrance du brevet: **25.07.2018**
(21) Numéro de dépôt: 13706649.4
(22) Date de dépôt: 07.02.2013
(51) Int. Cl.: A61F 2/16, C08L 23/12, C08L 33/04, C08L 39/06, C08L 75/04, C08L 77/12

(54) **COMPOSITION POLYMERES METASTABLES POUR DISPOSITIFS D'INJECTION D'IMPLANTS OPHTALMIQUES**
METASTABILE POLYMERZUSAMMENSETZUNGEN FÜR VORRICHTUNGEN ZUR INJEKTION VON AUGENIMPLANTATEN
METASTABLE POLYMER COMPOSITONS FOR DEVICES FOR INJECTION OF OPHTHALMIC IMPLANTS

(30) Priorité: 07.02.2012 FR 1251146; 16.05.2012 US 201261647751 P
(43) Date de publication de la demande: 17.12.2014
(73) Titulaire: Polymerexpert SA, 33600 Pessac (FR)
(72) Inventeur: DOLATKHANI, Marc, F-33610 Cestas (FR); HUPIN, Christophe, F-33770 Salles (FR)
(74) Mandataire: Santarelli
(86) Numéro de dépôt international: PCT/FR2013/050258
(87) Numéro de publication internationale: WO 2013/117863

(56) Documents cités:
- WO-A2-2006/128795
- Ä DEG LHAN Ã ZEN ET AL: "Modification of surface properties of polypropylene films by blending with poly(ethylene--ethylene oxide) and its application", POLYMER BULLETIN, SPRINGER, BERLIN, DE, vol. 68, no. 2, 21 octobre 2011 (2011-10-21), pages 575-595, XP019988683, ISSN: 1436-2449, DOI: 10.1007/S00289-011-0655-0
- XU X ET AL: "PROGRAMMABLE DRUG DELIVERY FROM AN ERODIBLE ASSOCIATION POLYMER SYSTEM", PHARMACEUTICAL RESEARCH, SPRINGER NEW YORK LLC, US, vol. 10, no. 8, 1 August 1993 (1993-08-01) , pages 1144-1152, XP000972186, ISSN: 0724-8741, DOI: 10.1023/A:1018960016756

## Description

L'invention concerne de nouvelles compositions polymères dans un état morphologique métastable capables de conduire, directement, à l'issue de leur mise en oeuvre, à des dispositifs biomédicaux possédant à leur surface des propriétés de glissant. L'invention concerne lesdites compositions polymériques métastables, leur utilisation pour la fabrication de dispositifs ou éléments de dispositifs présentant une surface glissante, les dispositifs à surface glissante issus de ces compositions polymères métastables et leurs applications, notamment dans le domaine des injecteurs ophtalmiques.

Le remplacement du cristallin de l'oeil affecté par la cataracte est assuré par des implants intraoculaires. La chirurgie utilisant la phacoémulsification permet la destruction du cristallin naturel et son élimination par une petite incision. Il a été développé des implants en matériaux souples et pliables pouvant être insérés à l'aide de dispositifs d'injection à travers la micro-incision réalisée pour la phacoémulsification.

Le système d'injection est composé d'un corps tubulaire dans lequel coulisse le piston d'injection surmonté d'une cartouche de chargement de l'implant ophtalmique et d'un embout de forme conique dont le diamètre diminue au fur et à mesure que l'on se rapproche de l'extrémité d'injection. Il existe deux types de système d'injection :
- l'injecteur monobloc dans lequel la cartouche de chargement de l'implant et l'embout sont liés au corps tubulaire de l'injecteur.
- l'injecteur constitué de deux pièces et dans lequel la cartouche de chargement de l'implant et l'embout sont séparés du corps tubulaire. Les deux pièces s'emboîtent après le chargement de l'implant dans la cartouche.

Quel que soit le type d'injecteur retenu, le chirurgien appuie sur le piston dont l'extrémité pousse l'implant qui se trouve dans la cartouche ; ce dernier est contraint de plus en plus dans l'embout de l'injecteur et finit par ressortir de l'injecteur totalement plié. Il est ainsi possible d'injecter un implant de plus de 6mm de diamètre par une incision de taille inférieure à 3mm. De très grandes contraintes sont alors exercées sur l'implant lors du transfert. Pour limiter la force d'injection et permettre à l'implant de sortir de l'embout sans dommages, il est nécessaire, d'une part, d'optimiser la géométrie de l'injecteur et, d'autre part, d'utiliser un « lubrifiant ».

Les demandes de brevet EP 1 173 115, EP 2 344 073 ou bien WO2007/054644 et WO2007/021412, décrivent des systèmes d'injection avec des conceptions géométriques différentes ; de nombreux injecteurs faisant appel aux différentes formes décrites dans ces demandes de brevets sont commercialement disponibles.

Les caractéristiques mécaniques du matériau constitutif de l'embout ont également une influence sur l'injectabilité. En effet, le matériau thermoplastique utilisé pour la fabrication de l'embout doit permettre une certaine déformabilité pour accompagner les contraintes infligées à l'implant tout en présentant une bonne rigidité. De plus, les matériaux thermoplastiques doivent pouvoir être mis en oeuvre à des cadences élevées. Ces matériaux sont choisis, de préférence, dans la famille des polyoléfines et plus particulièrement le polypropylène, les polyamides, les polyuréthanes et les polyesters.

Le choix du matériau constitutif de l'embout et de la cartouche ainsi que l'optimisation de sa géométrie ne suffisent cependant pas pour injecter des implants via des micro-incisions de façon satisfaisante. Il est impératif d'utiliser un lubrifiant permettant à l'implant de glisser dans l'embout et la cartouche. Les approches permettant la lubrification, décrites dans la littérature, sont l'utilisation d'un agent de migration et la mise en oeuvre d'un revêtement.

La première voie concerne l'utilisation d'un agent de migration (en anglais « blooming agent ») intégré par mélange (ou, selon le terme usuel, « compoundage ») dans le polymère thermoplastique. Il s'agit généralement d'une molécule organique oléophile ou tensio-active de faible masse molaire, contenant 10 à 30 atomes de carbone par molécule, dérivée d'acide carboxylique et de type monostéarate de glycérol (GMS), monopalmitate de glycérol ou bien monooléate de glycérol etc... Mis en mélange avec le polypropylène ou bien le polyamide, ce type de molécule se répartit uniformément dans le support thermoplastique obtenu par injection et finit par migrer à la surface du support après plusieurs jours voire plusieurs semaines. Ce phénomène de migration est lié à la petite taille de la molécule tensioactive qui est mobile comparée aux chaînes macromoléculaires du matériau thermoplastique. A titre d'exemple, les brevets US 6 733 507, US 6 679 891 et US 6 083 230 décrivent des cartouches en polypropylène (PP) contenant un agent lubrifiant qui migre à la surface par un phénomène de migration (« blooming »). L'injection de l'implant est réalisée après addition d'un produit visqueux (solution aqueuse d'hyaluronate ou bien d'hydroxypropylméthyl cellulose).

Cette approche présente deux inconvénients majeurs.

Le premier est la présence de traces blanches sur les implants injectés. Elles sont dues au fait que l'agent de migration n'est pas lié à la surface de l'embout et est entraîné mécaniquement lors de l'injection. En effet, les agents de migration utilisés ne sont pas hydrosolubles et leur élimination une fois l'implant injecté n'est possible qu'après de nombreux rinçages.

Le second inconvénient est lié à la cinétique de migration de l'agent de migration à la surface du support. Cette phase peut durer plusieurs jours, voire plusieurs semaines en fonction des conditions de mise en oeuvre (injection-moulage de la pièce), de la température de stockage, de post-traitement, des conditions de stérilisation, etc..., avant qu'une quantité suffisante de lubrifiant se trouve à la surface de la cartouche d'injection. La qualité de la lubrification va donc dépendre du temps d'attente entre la fabrication de l'injecteur et l'utilisation de celui-ci par le praticien. Si cette période est trop courte, la lubrification n'est pas assurée de manière satisfaisante et si elle est trop longue, l'implant injecté est couvert de taches blanches (présence d'agent de migration).

Pour pallier cet inconvénient, la demande WO 2005/018505 propose de traiter thermiquement les pièces afin d'accélérer le phénomène de migration et d'obtenir un état quasi-stable (quantité de lubrifiant suffisante à la surface du support) permettant des injections reproductibles.

Une autre voie décrite par le brevet US 7 348 038 est un traitement plasma pour assurer une liaison physique de l'agent de migration à la cartouche.

La non-reproductibilité de la lubrification et la présence de taches blanchâtres sur les implants après injection représentent les principaux défauts de l'utilisation de ces agents de migration.

Une deuxième approche (approche « revêtement ») permettant le glissement des implants dans les embouts/cartouches des injecteurs est la mise en place d'un revêtement hydrophile à l'intérieur de l'embout. Le principe de lubrification consiste à gonfler le revêtement hydrophile par l'addition d'un produit visqueux (solution aqueuse d'hyaluronate ou bien d'hydroxypropyl-méthylcellulose) et à faire glisser l'implant sur un film d'eau formé à l'interface.

Les brevets ou demandes de brevet JP56090838, JP3254752, US5 716 364, EP1949871, WO96/22062, WO2007/030009, WO2010/118080, US7 687 097, US7 348 038 et WO2010/059655 décrivent la possibilité de réaliser un revêtement permettant de diminuer ou d'éliminer la friction entre l'implant et la cartouche.

Le revêtement hydrophile est lié soit par des liaisons chimiques covalentes soit par des liaisons physiques à la surface de l'embout/cartouche.

En ce qui concerne les revêtements liés par des interactions physiques au support, une activation de la surface par un plasma ou corona ou bien d'autres méthodes photochimiques, est systématiquement préconisée comme présenté dans les brevets ou demandes de brevet JP56090838, JP3254752, EP1949871, US5 716 364, WO2010/059655, WO2010/118080 ou bien US7 348 038. Une fois la surface activée, le polymère en solution est déposé et le solvant est ensuite évaporé.

Les polymères hydrophiles sont, par exemple, choisis parmi le polyacide acrylique, le polyacide méthacrylique, le polyvinylacétate, le polyacrylamide, la polyvinylpyrolidone, le polyuréthane et leurs copolymères, et parfois un mélange de plusieurs de ces polymères.

Il est par ailleurs décrit des revêtements liés de manière covalente à la cartouche et l'embout notamment dans les brevets ou demandes de brevet EP 0 804 129, EP 0 952 796, US7 687 097 et WO96/22062. L'approche préconisée notamment dans les documents US7 687 097 et WO96/22062 consiste à immerger la cartouche, pré-traitée ou non par plasma, dans un précurseur présentant des fonctions réactives, par exemple des groupements acrylates, puis ensuite, par voie radicalaire (thermique ou irradiation UV), amorcer la polymérisation du précurseur dont certaines chaînes viennent réagir avec les radicaux formés au niveau de la surface de la cartouche.

En ce qui concerne l'approche « revêtement », les propriétés glissantes sont constantes au cours du temps et n'évoluent généralement pas comme dans le cas des agents de migration.

L'inconvénient majeur des procédés qui consistent à revêtir les dispositifs réside dans la complexité d'élaboration de tels revêtements, en particulier lorsqu'il s'agit de la surface interne des injecteurs. En effet, cette approche nécessite d'opérer en plusieurs étapes : i) activation de la surface, ii), dépôt du film polymère ou polymérisation/greffage puis iii) évaporation du solvant ou du composé non réagi, enfin iv) contrôle des caractéristiques du film (homogénéité/ épaisseur), etc. Ces procédures augmentent de manière significative le temps de production et le coût d'accès à des dispositifs possédant une surface glissante.

D'autres brevets ou demandes de brevet comme EP 0910 311, EP 1 198 207, US6 283 975, US6 398 788, US6 733 507, US6 679 891 et US6 083 230 décrivent également la possibilité d'utiliser, comme agent de lubrification, des polymères hydrophiles. Sont notamment cités le polyéthylène glycol, la polyvinylpyrrolidone, le poly(N-vinyl lactame), le polyacide acrylique, le polyoxyde d'éthylène, le polyoxyde de propylène (mentionné par erreur comme un polymère hydrophile), la polyvinylpyridine (mentionné par erreur comme un polymère hydrophile), le polyalcool vinylique, les polysaccharides, le carboxyméthyl cellulose, les hydroxyalkyl celluloses, le polyacide méthacrylique, le polyacrylamide, les polypeptides, le polystyrène sulfonate de sodium, le polyhydroxyéthyl méthacrylate, l'héparine et leurs mélanges.

Aucune indication claire n'est cependant donnée sur la mise en oeuvre de ces systèmes de lubrification et sur leur pouvoir lubrifiant. En revanche, ces demandes mentionnent, d'une part, la présence du polymère lubrifiant à la surface ou proche de la surface du support et, d'autre part, des interactions de type physique entre le support et le polymère lubrifiant (en anglais « physically secured »).

L'inconvénient majeur de l'utilisation des polymères cités dans ces demandes de brevet est que les polymères hydrophiles utilisés se détachent du support dans de nombreux cas. Ils sont ainsi entraînés dans l'oeil, les interactions de type physique avec le support n'étant pas suffisamment fortes pour empêcher la solubilisation du polymère hydrophile.

Un autre inconvénient de ces procédés est qu'ils nécessitent un post traitement de la pièce du dispositif une fois élaborée, pour apporter la lubrification recherchée (post-traitement thermique, traitement plasma, dépôt d'un revêtement), rendant l'élaboration du dispositif glissant plus onéreux.

L'article Ozen et al., Polym. Bull., 2012, 68, 575-595 rapporte l'étude de la compatibilité et de la migration d'un copolymère diblocs poly(éthylène-oxyde d'éthylène) amphiphile, de masse molaire 2250 g/mole, contenant 79% massique d'oxyde d'éthylène, dans un copolymère polypropylène-co-éthylène (3,6% massique d'éthylène). L'objectif est d'accroître l'adhésion entre les couches de polypropylène (PP) et de polyamide (PA) dans des films multicouches, grâce à la migration du copolymère amphiphile aux interfaces. La migration étudiée sur des systèmes modèles (films obtenus par évaporation d'un solvant, par extrusion ou par extrusion soufflage) est observée uniquement lorsque la surface de PP est en contact avec une plaque de verre, utilisée comme modèle d'une couche de PA.

Le problème technique à résoudre est donc de fournir un matériau comprenant un mélange de polymères permettant de conférer à la fois les propriétés mécaniques et les propriétés de lubrification souhaitées, pour l'élaboration d'injecteurs ophtalmiques et plus particulièrement celui de l'embout/cartouche, afin que ceux-ci-présentent des qualités de glisse et de stabilité susceptibles de conférer des propriétés comparables aux cartouches/embouts revêtu(e)s, tout en réduisant le nombre d'étapes, et donc les coûts, de fabrication. La solution devra également tenir compte de la création d'interactions fortes entre le matériau constitutif et le polymère permettant la lubrification, pour éviter ou du moins limiter toute solubilisation et/ou entraînement du polymère lubrifiant dans l'oeil du patient.

Dans la suite de la description, on utilisera indifféremment les termes « injecteur ophtalmique » ou « dispositif pour l'injection d'implant ophtalmique ».

On utilisera également indifféremment les termes « glisse » ou « glissement ».

La présente invention a pour objet, notamment, d'apporter directement les propriétés de glisse requises à des dispositifs pour l'injection d'implants ophtalmiques (ou lentilles intraoculaires) par micro-incision durant la phase de mise en oeuvre (élaboration par injection/moulage). L'invention permet également de remédier aux inconvénients et défauts induits par les différents procédés de post-traitements décrits précédemment. Ces objectifs sont atteints par l'utilisation de nouvelles compositions polymères constituées par des mélanges de polymères et de copolymères ayant des caractéristiques particulières et possédant une morphologie métastable apte à conduire, directement, lors de la mise en oeuvre des dispositifs, à des propriétés de glisse permettant l'injection de lentilles intraoculaires par micro-incision.

L'invention a donc pour objet ces compositions polymériques métastables particulières, le procédé d'élaboration des dispositifs ou éléments de dispositifs à surface glissante, les dispositifs à surface glissante issus de ces compositions métastables et leurs applications comme systèmes d'injection de lentilles intraoculaires par micro-incision de 3 mm ou moins.

On entend par « compositions polymères métastables » des mélanges de deux ou plusieurs polymères ou copolymères partiellement miscibles ou compatibles (comme définit par l'IUPAC dans la revue Pure Appl. Chem., Vol 76, n°11, pp 1985-2007, 2004) aptes à conduire à des matériaux polymères de morphologie multiphasique plus stable et qui sont bloqués dans une structure morphologique métastable.

On entend par « morphologie métastable » un système hors de son état d'équilibre morphologique et dont la cinétique d'évolution vers sa forme thermodynamiquement stable est extrêmement lente dans des conditions données et peut être négligée dans une échelle de temps déterminée.

Cette évolution dans l'organisation morphologique peut toutefois être provoquée par apport d'énergie au système, comme par exemple par une élévation suffisante de sa température pendant un temps limité. Dans le cas particulier d'un mélange de polymères partiellement miscibles ou compatibles la morphologie sera dite métastable lorsque les processus de ségrégation des phases par migration des chaînes polymères ou copolymères ne seront pas achevés et n'évolueront pas ou seulement de façon négligeable dans des conditions de température données et sur une échelle de temps déterminée.

Sont inclus dans cette définition des mélanges présentant des morphologies d'apparence monophasique, correspondant à une répartition macroscopiquement uniforme des différents composants polymères ainsi que des morphologies bi- ou multiphasiques transitoires, qui vont évoluer lorsque l'on élève suffisamment la température desdits mélanges.

Il est bien connu, par ailleurs, que si la mobilité des chaînes polymères dépend de la température, elle dépend également de l'état physique dans lequel se trouvent lesdits polymères.

Pour un polymère amorphe la mobilité des chaînes va dépendre en particulier de sa température de transition vitreuse (Tg), correspondant au passage de l'état vitreux à l'état visco-élastique.

Pour un polymère cristallin ou semi cristallin, la mobilité sera déterminée par la température de fusion (Tf).

Dans le cas d'un mélange de polymères partiellement miscibles ou compatibles, en dessous de la Tg/Tf de chacun des constituants la mobilité des chaînes est fortement réduite empêchant sur une très grande échelle de temps tout réarrangement morphologique tel que séparation de phases. Plus l'on descend en dessous des Tg(s)/Tf(s), plus le système est immobile, figé. A l'inverse, au dessus des Tg(s)/Tf(s), les chaînes acquièrent une mobilité permettant leur migration au sein du matériau, faisant évoluer sa morphologie vers une organisation multiphasique thermodynamiquement plus stable.

Si l'on exclut le cas de certains polymères qui deviennent miscibles à haute température (systèmes présentant une température critique supérieure de solubilité), plus on élèvera la température au dessus des Tg(s)/Tf(s), plus la mobilité des chaînes sera grande et plus la cinétique de réorganisation morphologique du mélange sera rapide.

Pour un polymère dans un mélange de polymères partiellement miscibles ou compatibles donné, la diffusion/migration des chaînes est également dépendante de sa masse molaire; la cinétique de migration sera d'autant plus rapide que les chaînes possèdent des masses molaires faibles.

Enfin, la migration des chaînes polymères dans un mélange dépend du caractère de compatibilité/miscibilité entre les dites chaînes, elle ralentit lorsque la compatibilité entre polymères augmente. Une miscibilité partielle, tel que décrit dans la demande WO2009/156828, conduit, en plus de la séparation de phases, à la création de micro-domaines caractérisés par des températures de transition propres où les différentes chaînes sont en interaction moléculaire étroite. Ces micro-domaines permettent d'établir des liens entre les phases par enchevêtrement des chaînes. Ces liens ralentissent la diffusion/migration des chaînes aux alentours des Tg(s)/Tf(s) et renforcent à ces températures le caractère figé des compositions métastables. Ils ont cependant des effets négligeables sur la diffusion aux températures très supérieures aux Tg(s)/Tf(s) des différentes phases et micro-domaines.

Les compositions métastables selon l'invention sont obtenues à partir de mélanges de polymères et de copolymères partiellement miscibles ou compatibles. Ces mélanges sont caractérisés par une morphologie bi ou multiphasique, et par la présence d'interphases constituées par des chaînes du polymère constitutif et des séquences ou blocs hydrophobes du copolymère de fonction. Les polymères et copolymères de l'invention présentent en outre certaines caractéristiques spécifiques qui sont indiquées ci-après. Les mélanges correspondants, obtenus par compoundage, sont caractérisés par un état morphologique métastable. Ils sont composés en particulier :
1) d'au moins un polymère thermoplastique formant la structure et conférant les propriétés mécaniques, dit « polymère constitutif ».
   Il présente une masse molaire élevée, notamment supérieure à 40000 g/mole, de préférence supérieure à 100 000 g/mole, et une Tg ou Tf élevée, supérieure ou égale à 80°C, et de préférence supérieure ou égale à 100°C.
   Ce premier polymère, qui a pour rôle d'apporter les caractéristiques mécaniques au matériau et aux pièces ou dispositifs préparés à partir dudit mélange, est en proportion massique majoritaire, à savoir en proportion massique comprise entre 85% et 99,9% et plus particulièrement entre 90% et 99,5% massique.
   Ce polymère constitutif est choisi, de préférence, parmi les polyoléfines, et plus particulièrement le polypropylène, les polyamides, les polyuréthanes et les polyesters.
   Ces polymères constitutifs sont mis en oeuvre par injection/moulage à des températures comprises entre 160°C et 300°C.
2) au moins un copolymère dit « de fonction », partiellement miscible ou compatible avec le polymère constitutif mentionné ci-dessus; le caractère partiellement miscible ou compatible étant assuré par des séquences ou blocs de co-monomères à caractère hydrophobe choisis.

Avantageusement, ledit polymère constitutif apporte les propriétés mécaniques et ledit copolymère de fonction apporte un caractère glissant au matériau élaboré à partir dudit mélange, notamment lorsqu'il est mis en présence d'une solution ou suspension aqueuse.

Les polymères de fonction ont pour rôle d'apporter au matériau et aux pièces ou dispositifs élaborés à partir dudit mélange des propriétés spécifiques, notamment des propriétés de surface. Ainsi les propriétés de glissant recherchées pour les dispositifs d'injection de lentilles intraoculaires sont obtenues préférentiellement avec des copolymères possédant majoritairement des blocs ou séquences à caractère hydrophile et de façon minoritaire des séquences ou blocs hydrophobes apportant une compatibilité partielle avec le premier polymère ; les blocs hydrophiles représentant plus de 60% du copolymère.

Les propriétés de glissant vont s'exprimer par ajout dans le dispositif d'injection d'une solution ou suspension aqueuse, dans laquelle baigne la lentille intraoculaire, et qui forme une couche superficielle d'eau stabilisée par hydratation des blocs hydrophile du copolymère de fonction localisés en surface du matériau formé à partir de la composition selon l'invention. Cette interface liquide entre ledit matériau et la lentille intraoculaire va fortement réduire les phénomènes de friction et apporter le caractère glissant permettant l'injection sous faible contrainte de la lentille à travers l'embout de faible diamètre du dispositif d'injection.

En particulier, la lentille intraoculaire peut être introduite en présence d'une solution aqueuse d'acide hyaluronique, de hyaluronate, d'hydroxypropylmethylcellulose ou de tout autre agent visqueux hydrophile.

Cette solution est ajoutée entre 30 secondes et 5 minutes environ avant l'injection de l'implant par la micro-incision, comme indiqué précédemment.

Le « caractère glissant » du matériau élaboré à partir de la composition métastable décrite plus haut peut être mesuré, par exemple, en utilisant un injecteur ophtalmique monobloc entièrement fabriqué par injection/moulage à partir de ladite composition ou bien un injecteur constitué de deux pièces dans lequel seuls la cartouche de chargement et l'embout sont fabriqués à partir de ladite composition. On mesure la force de poussée sur le piston de l'injecteur nécessaire pour éjecter l'implant chargé dans la cartouche de l'injecteur, à travers un embout dont le diamètre de sortie est inférieur à 3 mm, par exemple 2 mm. Cette mesure peut être effectuée par compression au moyen d'un dynamomètre de type Instron 3367 équipé d'un capteur de force de sensibilité 0,5 kN à une vitesse de 8,5 mm/s. On considère que la propriété de glissement est importante et non traumatisante pour des valeurs de force comprises entre 1 et 5 N., Elle est modérée pour des valeurs de force supérieures à 5 N et inférieures à 10 N, et faible pour des valeurs de force supérieures à 10 N .

Ainsi, on considère qu'un matériau élaboré à partir de la composition métastable selon l'invention confère à un injecteur ophtalmique un « caractère glissant » lorsqu'un praticien injecte sans dommage un implant d'une dioptrie inférieure ou égale à 30D dans l'oeil d'un patient, en appliquant une force inférieure ou égale à 5N.

La masse molaire ainsi que les dimensions relatives des séquences ou blocs qui contribuent à la balance hydrophile/hydrophobe des copolymères sont de préférence choisies de façon, d'une part, à optimiser leur compatibilité partielle avec le matériau à base du polymère constitutif (par exemple, PP ou PA) et, d'autre part, à permettre leur migration et leur interaction avec le milieu aqueux extérieur, conférant ainsi à la surface du matériau un caractère hydrophile favorisant la création d'une couche d'eau superficielle, tandis que les interactions des séquences ou blocs hydrophobes du copolymère de fonction avec le matériau à base du polymère constitutif empêchent une éventuelle solubilisation du copolymère dans la solution aqueuse.

Une estimation de la balance hydrophile/hydrophobe des copolymères peut-être réalisée par l'étude de la micellisation des copolymères dans l'eau, ceux conduisant à des micelles stables possédant une structure appropriée permettant de satisfaire aux caractéristiques de surface et de glissant recherchées.

Les copolymères de fonction selon l'invention possèdent des masses molaires supérieures à 5000 g/mol, de préférence comprises entre 10000 et 100000 g/mole, et en particulier de préférence entre 10000 et 40000 g/mol. Le choix de copolymères de masse molaire supérieure à 5000 g/mol permet de limiter leur solubilisation dans la phase aqueuse. Ces copolymères, de fonction doivent en outre posséder des Tg(s) ou Tf(s) comprises dans une gamme de températures supérieures ou égales à 40°C, et inférieures aux valeurs de Tg ou Tf des polymères constitutifs. Ils doivent également posséder une stabilité thermique, à la température de mise en oeuvre du polymère constitutif, généralement comprise entre 160°C et 300°C, leur permettant de supporter les conditions de mise en oeuvre par injection-moulage sans dégradation chimique ou structurale.

Les copolymères de fonction qui apportent des propriétés de glissant peuvent être choisis parmi les copolymères statistiques, les copolymères à blocs, les copolymères en peigne, les copolymères en étoile. Ils peuvent être, de préférence, choisis parmi les polyéthers, les polyesters et les polyuréthanes présentant à la fois des séquences ou blocs hydrophiles et hydrophobes, et, parmi les copolymères à base d'acide (méth)acrylique, de (méth)acrylate, d'oxyde d'éthylène, d'acrylamide, d'alcool vinylique, de vinyl pyrolidone ou d'hydroxyéthyl (méth)acrylate associés à des motifs hydrophobes partiellement miscibles ou compatibles avec le polymère constitutif.

En outre, ces copolymères de fonction présentent une partie hydrophile majoritaire, à savoir au moins 60% massique d'unités monomères hydrophiles, notamment entre 60 et 99% massique d'unités monomères hydrophiles et en particulier entre 70 et 97% massique d'unités monomères hydrophiles.

Quant aux séquences ou blocs minoritaires hydrophobes, leur rôle est, d'une part, de limiter les risques de solubilisation en milieu aqueux du copolymère et, d'autre part, d'apporter une miscibilité partielle ou compatibilité avec le polymère constitutif.

On peut citer, à titre d'exemple, l'existence d'une compatibilité partielle entre les blocs de polyoxyde de propylène et le polypropylène, ou bien entre les séquences de polycaprolactone et le polypropylène, ou enfin entre des séquences de polyester et le polyamide.

On peut également utiliser un copolymère de fonction présentant, en plus des caractéristiques mentionnées plus haut, des groupements réactifs capables de réagir avec le polymère thermoplastique au cours de l'opération de mise en oeuvre par injection/moulage.

Dans ce cas, les copolymères de fonction sont dotés de groupements réactifs qui, par exemple, vont réagir par voie radicalaire (thermique ou bien photochimique) avec les radicaux créés sur les chaînes du polymère constitutif.

Les compositions polymères métastables selon l'invention sont préparées par compoundage et extrusion afin d'obtenir ces compositions sous diverses formes : billes, granules, etc., pouvant être directement mises en oeuvre par injection/moulage, pour réaliser en une seule étape des dispositifs possédant les propriétés de glissant recherchées.

Ces compositions métastables présentent l'avantage et la particularité d'être selon les conditions de température :
- soit dans un état figé métastable dans lequel elles peuvent être conservées sans évolution de leur morphologie pour une durée de plusieurs mois ou même de plusieurs années. Cet état correspond au domaine des températures inférieures aux Tg/Tf des différents composants polymère du mélange.
- soit dans un état «activé», c'est à dire mises en condition d'évoluer rapidement, vers une morphologie multiphasique, thermodynamiquement plus stable. Cet état activé correspond aux températures élevées, supérieures aux Tg/Tf des constituants polymères, températures pour lesquelles la mobilité des chaînes est optimale.

Dans l'invention, cette activation/évolution est réalisée durant le temps de mise en oeuvre des dispositifs à haute température, typiquement quelques secondes à quelques minutes à des températures d'injection-moulage comprises entre 160°C et 300°C. Il résulte directement de cette opération un dispositif réalisé en un matériau multiphasique, caractérisé en ce que la matrice comprend ou est constituée par le premier polymère, dit polymère constitutif, tandis que les seconds polymères, dits de fonction, forment des domaines distribués de façon non homogène entre le coeur et la surface du matériau, apportant en particulier les propriétés de surface telle que la glisse au dispositif. L'invention concerne également les matériaux, pièces ou dispositifs obtenus à partir des compositions polymères métastables décrites plus haut, qui, de manière avantageuse, possèdent en présence d'eau la propriété de surface requise, notamment le caractère glissant. Avantageusement, lesdits matériaux, pièces ou dispositifs, peuvent être réalisés en une étape par injection/moulage à partir de compositions polymères métastables décrites plus haut.

Un autre avantage de l'invention est l'obtention d'une propriété de surface permettant d'obtenir des caractéristiques de glisse qui demeurent stable et constante sur une grande échelle de temps,, depuis l'élaboration des pièces ou dispositifs par injection/moulage jusqu'à l'utilisation du dispositif par le praticien. Le caractère glissant est obtenu à tout moment par simple ajout d'une solution d'agent visqueux. Cette propriété de surface est maintenue, à la simple condition de conserver la pièce ou le dispositif à des températures inférieures aux températures de transition vitreuse ou de fusion des différents composants polymères, ce qui est aisément réalisé lorsque les polymères et copolymères utilisés ont des Tg ou Tf supérieures aux températures usuelles de stockage des dispositifs, températures qui sont très généralement inférieures à 50°C, de préférence en dessous de 40°C.

Un autre avantage de l'invention résulte de l'utilisation de copolymères de fonction possédant à la fois des motifs hydrophiles et des motifs hydrophobes. L'optimisation de la balance hydrophile/hydrophobe du copolymère permet d'éviter sa solubilisation en milieu aqueux. Ce caractère est renforcé par la miscibilité des séquences hydrophobes avec le polymère constitutif, ce qui limite la diffusion/migration aux températures de stockage et d'utilisation des pièces ou dispositifs, sans pour autant affecter la séparation de phases qui a lieu durant la mise en oeuvre par injection-moulage de la pièce du dispositif. Ces caractéristiques assurent une grande stabilité dans le temps au système et limite tout entraînement du copolymère de fonction lors de l'injection de l'implant par le praticien.

Selon un aspect particulier de l'invention, un ancrage covalent entre le polymère thermoplastique constitutif et le copolymère de fonction hydrophile peut être réalisé durant le procédé d'injection/moulage à l'aide de groupements réactifs fixés sur le copolymère de fonction. Ceci présente l'avantage particulier d'obtenir un dispositif ayant une surface glissante encore plus stable en milieu aqueux. L'intérêt de cette approche concerne en particulier les systèmes d'injection contenant des implants pré-chargés dans une cartouche contenant un liquide physiologique.

Pour obtenir un greffage permanent, on peut utiliser un copolymère de fonction présentant, en plus des caractéristiques mentionnées plus haut, des groupements réactifs capables de réagir avec le polymère thermoplastique au cours de l'opération de mise en oeuvre par injection/moulage.

Pour ce mode particulier, les copolymères de fonction sont dotés de groupements réactifs qui, par exemple, vont réagir par voie radicalaire (thermique ou bien photochimique) avec les radicaux créés sur les chaînes du polymère constitutif. Ces groupements réactifs peuvent, par exemple, être des fonctions méth(acrylate) capables de réagir en présence d'un amorceur radicalaire au cours de la mise en oeuvre à plus de 160°C, procédé qui s'apparente à de l'injection/moulage réactive. L'amorceur qui va permettre d'initier la polymérisation radicalaire peut être introduit dans l'extrudeuse avec la composition métastable.

Dans certains cas, il peut être préférable de procéder à ces réactions de couplage covalent entre le copolymère de fonction et le polymère constitutif, après que la ségrégation de phase au sein du mélange soit réalisée. On peut utiliser dans ce cas une extrudeuse à deux entrées, ce qui permet d'introduire, dans un premier temps, la composition métastable puis, ultérieurement, le dérivé générateur de radicaux.

Une autre approche pour cet aspect particulier de l'invention, consiste à utiliser un photoamorceur. Celui-ci peut être introduit en même temps que la composition métastable constituée du polymère constitutif (par exemple, polypropylène) et du copolymère de fonction présentant des groupes méth(acryliques). L'opération de mise en oeuvre est alors accompagnée lors du démoulage d'une irradiation (UV, gamma) du dispositif provoquant l'ancrage photochimique du polymère glissant.

L'invention concerne également les matériaux, pièces ou dispositifs élaborés à partir des compositions polymères métastables décrites précédemment, en particulier préparés par injection/moulage, tels que, notamment, une pièce de dispositif médical ou un dispositif médical. En **particulier,** ladite pièce est tout ou partie d'un dispositif d'injection d'implant ophtalmique (injecteur ophtalmique), par exemple un embout ou une cartouche, ou une pièce embout/cartouche d'un injecteur ophtalmique.

L'invention concerne également les dispositifs d'injection d'implant ophtalmique comprenant au moins une pièce préparée à partir d'une composition métastable décrite plus haut.

L'invention concerne également un procédé de fabrication d'un matériau, d'une pièce de dispositif médical ou d'un dispositif médical, qui comprend une étape d'injection/moulage d'une composition métastable telle que décrite plus haut.

La présente invention diffère significativement de l'art antérieur décrivant l'utilisation d'agents de migration (« blooming agents ») par les points suivants :
- Les agents de migration sont des molécules organiques de masses molaires faibles de nature lipophile et/ou tensio-active, alors que, selon l'invention on utilise des copolymères de masses molaires élevées, de préférence supérieures à 5 000g/mol, capables de former un matériau de morphologie multiphasique.
- Les agents de migration migrent à la surface du matériau constitutif au cours du temps, ce qui signifie que les propriétés de glisse évoluent en fonction de la durée de stockage et d'utilisation du dispositif par le praticien. Elles dépendent également d'éventuels post-traitements thermiques, du mode de stérilisation, etc... Au contraire, selon l'invention, la morphologie multiphasique est obtenue au moment de la fabrication de l'injecteur monobloc ou bien de l'embout/cartouche des injecteurs composés de deux pièces. Les propriétés de glisse peuvent de ce fait être générées par simple ajout d'une solution aqueuse sitôt après la mise en oeuvre tandis que, pour les raisons évoquées précédemment, elles n'évoluent pas au cours du temps.
   - Les agents de migration en surface, qui ne sont pas liés au polymère thermoplastique constitutif, sont entraînés dans l'oeil lors de l'injection de l'implant. Leur caractère lipophile et/ou tensio-actif les rend insolubles dans l'eau et conduit à leur dépôt sur la surface de l'implant sous la forme de traces blanches. Ces dépôts doivent être absolument éliminés au risque de perturber la qualité optique de l'implant mis en place, problème absent dans le cas de la présente invention.

La présente invention diffère également totalement de l'approche « revêtement » (« coating »). Cette dernière, qui consiste à déposer un film polymère à la surface du dispositif ou de certains de ses éléments, nécessite de réaliser plusieurs opérations dont l'activation de la surface, la dépose du polymère hydrophile en solution et l'évaporation du solvant L'approche proposée dans la présente invention, permet d'éviter toutes ces étapes dans le processus de fabrication et conduit à une simplification du procédé à la seule étape de mise en oeuvre par injection.

Enfin, la présente invention diffère de l'art antérieur qui consiste à associer, par simples interactions physiques, le polymère constitutif à des homopolymères hydrophiles, voire hydrosolubles en tant qu'agent lubrifiant. L'invention se distingue, d'une part, par la nature des copolymères utilisés qui possèdent à la fois des séquences à caractère hydrophile et des séquences hydrophobes et dont la balance hydrophile/hydrophobe permet de limiter la solubilisation lors de l'injection. L'invention se distingue d'autre part, par le caractère partiellement miscible ou compatible des séquences ou blocs hydrophobes des copolymères de fonction avec le polymère constitutif ce qui conduit à la formation de micro-domaines communs qui assurent un lien entre les phases dans les conditions de stockage et d'utilisation. Ces caractéristiques des copolymères de l'invention apportent une stabilité des propriétés de glisse et évitent tout entraînement dans l'oeil du patient.

Les exemples qui suivent, illustrent l'invention de manière non limitative.

Les exemples 1 à 5 concernent la préparation de copolymères de fonction permettant d'obtenir, en mélange avec un polymère constitutif, une composition métastable selon l'invention.

Les exemples 6 et 7 concernent la préparation de copolymères de fonction possédant des groupes réactifs permettant d'obtenir, en mélange avec un polymère constitutif, une composition métastable selon l'invention et présentant en plus la particularité de pouvoir se greffer chimiquement sur le polymère constitutif.

Les exemples 8 à 13 concernent l'élaboration des compositions métastables selon l'invention, leur utilisation pour la fabrication de cartouches d'injecteurs ophtalmiques et la caractérisation des propriétés de glisse de ces injecteurs pour l'injection d'implants ophtalmiques.

Exemple 1 : préparation d'un polyuréthane urée à base de copolymères triblocs poly (oxyde d'éthylène-b-oxyde de propylène-b-oxyde d'éthylène) (POE-POP-POE) terminé par des motifs acide.

La synthèse s'effectue en deux étapes. Dans une première étape, 3,8.10⁻³ mole de polymère F1 27 Pluronic® non séché contenant 0,3% en masse d'eau est dissous dans 150 ml de 2-butanone. Ensuite, 1,5.10⁻² mole de 4,4'-méthylène biscyclohexyl di-isocyanate sont ajoutés goutte à goutte durant 10 minutes sous flux continu d'azote. Lorsque environ 81% des fonctions isocyanate ont été consommées (suivi par spectroscopie infra-rouge à transformée de Fourier), 2,9.10⁻³ mole d'acide 2,2-(bishydroxymethyl) butyrique sont ajoutés. La polycondensation se poursuit pendant 2 heures à 70°C jusqu'à la disparition complète des fonctions isocyanate. Le copolymère est recueilli par précipitation dans l'éther diéthylique et séché sous vide à température ambiante.
Le copolymère possède une température de fusion de 52°C. L'analyse par chromatographie d'exclusion stérique (CES) indique la présence de chaînes de copolymères de masses molaires supérieures à 10 000 g/mol (étalonnage Polystyrène).

Exemple 2 : préparation d'un polyuréthane urée à base de copolymère POE-POP-POE terminé à ses extrémités par des chaînes de POE.

7,69.10⁻³ mole de Pluronic F127 sont séchés sous vide à 80°C pendant 2 heures puis solubilisés dans 300 ml de 2-butanone contenant 0,2 g d'eau. 15,84.10⁻³ mole de 4,4' méthylène biscyclohexyl di-isocyanate sont ensuite ajoutés goutte à goutte durant 10 minutes sous flux continu d'azote. 500 ppm d'un catalyseur à base d'étain sont introduits après 30 minutes suivant l'addition de l'isocyanate. 13,98.10⁻³ mole d'un poly(oxyde d'éthylène) monohydroxylé de masse molaire égale à 600 g/mol est introduit lorsque 58% des fonctions iscyanate ont disparu (suivi par spectroscopie infra-rouge à transformée de Fourier). La réaction est poursuivie jusqu'à disparition totale des fonctions isocyanate et peut durer jusqu'à 12 heures. Le copolymère est ensuite précipité dans un non-solvant (éther de pétrole) puis séché sous vide à 40°C, Le copolymère possède une température de fusion de 52°C. L'analyse par CES (étalonnage polystyrène) indique la présence de chaînes de copolymères de masses molaires supérieures à 10 000 g/mol.

Exemple 3 : préparation d'un copolyuréthane à base de polyoxyde d'éthylène et de poly(ε-caprolactone).

400 g d'un POE dihydroxytéléchélique de masse molaire 6 000 g/mol (6,67.10⁻² mole) et 100 g d'un poly(ε-caprolactone) (PCL) dihydroxytéléchélique (8.10⁻² mole) de masse molaire 1 250 g/mol, sont séchés sous vide pendant 2 heures à 100°C puis solubilisés dans 1,45 litres de 2-butanone préalablement séchée sur CaCl₂. La solution est refroidie à 85°C au moment de l'introduction de 38,43 g (14,67.10⁻² mole) de di-isocyanate Desmodur W commercialisé par Bayer. 0,5 g d'un catalyseur à base de Bismuth est ajouté 5 minutes après la fin de l'introduction de l'isocyanate.

La réaction est terminée après 19 heures par l'addition d'1 ml d'éthanol. La solution est ensuite refroidie à température ambiante puis diluée dans 1,5 litre d'acétone puis précipitée dans 8 litres d'heptane.
La masse molaire en nombre du polymère obtenue (équivalent PS) est de 25 000g/mol. Le copolymère de type multiblocs présente une température de fusion de blocs s'étalant de 40 à 53°C.

Exemple 4: préparation d'un copolymère statistique de poly(méthacrylate de méthyle-co-méthacrylate de polyoxyde d'éthylène).

200 g de méthacrylate de polyéthylène glycol (MAPEG masse molaire 1 100 g/mol) sont solubilisés dans 800 ml de 2-butanone à température ambiante durant 1h30. 1,6 g de 2,2'-azobis 2-méthylbutane nitrile (amorceur radicalaire) est ajouté dans le milieu qui est ensuite porté à 100°C juste avant l'ajout de 20 g de méthacrylate de méthyle (MMA). Le milieu réactionnel est laissé à 100°C pendant 4 heures, puis est précipité dans l'heptane et séché sous vide à 40°C. La masse molaire en nombre (équivalent PS) du polymère obtenu est de 31 000 g/mol. Il présente en particulier une Tf d'environ 50°C des blocs polyoxyde d'éthylène.

Exemple 5 : préparation d'un copolymère statistique de poly(vinylpyrolidone-co-méthacrylate de 2-hydroxyéthyle-co-méthacrylate de polyester).
a) Synthèse du méthacrylate de polyester (FTL11228) 200 g d'ε-caprolactone, 67,66 g de méthacrylate de 2-hydroxyéthyle et 0,759 g d'un catalyseur à base d'étain sont solubilisés dans 216 ml de toluène anhydre distillé. Le système est porté à 90°C pendant 18h. Le macromonomère récupéré, caractérisé par RMN du proton, possède une masse molaire de 562 g/mol.
b) Synthèse du copolymère statistique de poly(vinylpyrolidone-co-méthacrylate de 2-hydroxyéthyle-co-méthacrylate de polyester)
   1- Dans un réacteur équipé d'un réfrigérant avec bulleur sous léger flux d'azote, on introduit 66,53 g de 2-hydroxyethyl méthacrylate, 7,24g de méthacrylate de polyester FTL11228 et 1,18 litre d'éthanol technique. Ce premier mélange est appelé pied de cuve.
   2- On prépare à côté un second mélange constitué de 59,69 g de 1-vinyl 2-pyrrolidone, 28,96 g de méthacrylate de polyester FTL11228, 216 g de 2-hydroxyethyl méthacrylate, 19,29 g de dodécanethiol, 5,79 g d'AIBN et 415 ml d'éthanol technique. Ce mélange n°2 est laissé sous agitation jusqu'à solubilisation.
   3- Le mélange n°2 est coulé goutte à goutte en 60 minutes sur le pied de cuve porté à reflux.
   4- L'ensemble est laissé au reflux sous léger courant d'azote pendant 3h30 à partir de la fin de la coulée.
   5- Le milieu final est laissé à refroidir, reconcentré, précipité dans l'eau et lyophilisé.
Le copolymère obtenu présente une Tg de 63°C.
Les masses molaires déterminées par chromatographie d'exclusion stérique (solvant DMF/LiBr - Colonne DMFext- Etalonnage PS) du copolymère sont Mw=22 000 g/mol ; Mp=21 420 g/mol ; Mn=13 900g/mol ; et l'indice de polydispersité est de 1,6.

Exemple 6 : préparation d'un copolymère polyuréthane à base de POE et de PCL terminé par des motifs acrylate à ses deux extrémités.

400 g d'un POE dihydrotéléchélique de masse molaire = 6 000 g/mol (6,67.10⁻² mole) et 100 g d'un poly(ε-caprolactone) (PCL) dihydroxytéléchélique (8.10⁻² mole) de masse molaire 1250 g/mol sont séchés sous vide pendant 2 heures à 100°C puis solubilisés dans 1,45 l de 2-butanone séché. 34 g de Desmodur W (di-isocyanate) et 0,5g d'un catalyseur à base de bismuth sont ajoutés au milieu réactionnel qui est porté à 85°C.

Après consommation totale des isocyanates, le milieu réactionnel est refroidi et 2 g de chlorure d'acryloyle sont ajoutés au milieu réactionnel La réaction est arrêtée 2 heures après l'ajout du chlorure d'acryloyle. Le polymère est ensuite précipité dans l'éther. L'analyse par Spectroscopie Infrarouge à Transformée de Fourier (IRTF) montre la présence d'insaturations sur les chaînes polymères. Le copolymère de type multiblocs présente une température de fusion de blocs s'étalant de 40 à 53°C.

Exemple 7 : préparation d'un copolymère statistique de poly(vinylpyrolidone-co-méthacrylate de 2-hydroxyéthyle-co-méthacrylate de polyester) portant des groupements acrylates pendants.

100 g de copolymère de l'exemple 6 sont solubilisés dans 500ml de THF sec. 2 g de chlorure d'acryloyle sont ajoutés à la solution à température ambiante. Après 4 heures, la réaction est arrêtée et le copolymère précipité dans l'heptane. L'analyse IRTF montre la présence d'insaturations de type acrylique sur les chaînes copolymères.

Exemple 8 : Un polypropylène de référence PPR 10222 commercialisé par la société TOTAL est mélangé (ou « compoundé ») avec le copolymère décrit dans l'exemple 2 dans les conditions suivantes :
- extrudeuse bi-vis Ø26 mm L=50D
- Débit : 10 kg/h
- Vitesse des vis : 300 rpm
- Profil de température : (pied) 210-210-200-190-180-170-170-160-160-160 (filière) : programmé ainsi en raison de la très grande fluidité du compound obtenu. La température est baissée en filière au voisinage de la température de fusion du PP. Chaque bloc de température fait 5D de long.

Les 2 composants polymères sont mélangés et introduits via un doseur gravimétrique en pied d'extrudeuse. Le copolymère de l'exemple 2 est mélangé avec le polypropylène PPR 10222 à des pourcentages de 1, 2, 5 et 10%. Les différentes compositions métastables obtenues (« compounds ») sont récupérées en sortie d'extrudeuse sous forme de granulés et conservées à température ambiante.

Les différentes compositions métastables obtenues sont ensuite mises en oeuvre par injection/moulage pour fabriquer des cartouches/embouts d'injecteurs d'implants ophtalmiques de diamètre de sortie 2 mm.

Les propriétés de glisse ont été évaluées sur les injecteurs correspondants à intervalles réguliers, sur 2 mois après leur date de fabrication. Les essais ont été réalisées avec des implants hydrophiles en hydroxyéthylméthacrylate (HEMA) (teneur en eau 28%) de dioptrie 27D, après ajout d'une solution aqueuse de hyaluronate (0,1 à 0,2ml, HA à 2,1%). Des essais comparatifs ont été réalisés dans les mêmes conditions sur des cartouches/embouts en polypropylène (PPR10222) compoundé avec du monostéarate de glycérol (GMS).

Le caractère glissant a été déterminé à partir de la force de compression nécessaire à l'injection de l'implant à travers le diamètre de sortie de 2 mm. Ces mesures ont été effectuées au moyen d'un dynamomètre de type Instron 3367 équipé d'un capteur de force de sensibilité 0,5 kN, à une vitesse de compression de 8,5 mm/s. Les résultats sont classés en fonction de la force mesurée en 3 catégories : glissement faible, glissement modéré, glissement important. Les résultats sont rapportés dans le tableau 1 ci-après.

**Tableau 1**

| N° | Nature de la cartouche/emb out | Essai réalisé 1 jour après la mise en oeuvre* | Essai réalisé 7 jours après la mise en oeuvre* | Essai réalisé 15 jours après la mise en oeuvre* | Essai réalisé 1 mois après la mise en oeuvre* | Essai réalisé 2 mois après la mise en oeuvre* |
|---|---|---|---|---|---|---|
| 1.1 | PP+GMS | Glissement faible | Glissement modéré | Glissement important. Présence de traces blanches. | Glissement important. Présence de traces blanches. | Glissement important. Beaucoup de traces blanches. |
| 1.2 | PP+1% du copolymère de l'exemple 2 | Glissement modéré | Glissement modéré | Glissement modéré | Glissement modéré | Glissement modéré |
| 1.3 | PP+ 2% du copolymère de l'exemple 2 | Glissement important | Glissement important | Glissement important | Glissement important | Glissement important |
| 1.4 | PP+ 5% du copolymère de l'exemple 2 | Glissement important | Glissement important | Glissement important | Glissement important | Glissement important |
| 1.5 | PP+ 10% du copolymère de l'exemple 2 | Glissement important | Glissement important | Glissement important | Glissement important | Glissement important |

| | | | | | | |
|---|---|---|---|---|---|---|
| **Chaque essai est réalisé 3 fois pour valider le résultat obtenu* Glissement faible : pouvant entraîner une dégradation importante des implants (force appliquée >10N) Glissement modéré : nécessitant une force élevée pour l'injection avec le risque d'altération des implants (5N<force appliquée<10N) Glissement important : permettant une injection aisée sans risque d'altération des implants (1N<force appliquée<5N) | | | | | | |

Les résultats montrent que les embouts fabriqués à partir de la composition métastable (polypropylène (PP) + copolymère de l'exemple 2) présentent des propriétés de glisse constantes au cours du temps, ce qui n'est pas le cas des embouts contenant du GMS. Ce glissement est important pour des compositions métastables contenant plus de 1% du copolymère de l'exemple 2. Les embouts contenant 5 et 10 % de copolymère de fonction sont plus opaques que leurs homologues contenant 1 et 2 % du copolymère de l'exemple 2.

Pour toutes les cartouches/embouts élaborées à partir de compositions métastables (PP + polymère de l'exemple 2), aucune trace sur l'implant n'est observée après injection.

### Exemple 9

Les mêmes cartouches/embouts sont utilisés pour des tests d'injection d'un implant hydrophobe souple de dioptrie 25D.

Les résultats sont rapportés dans le tableau 2 ci-après.

**Tableau 2**

| N° | Nature de la cartouche/emb out | Essai réalisé 1 jour après la mise en oeuvre* | Essai réalisé 7 jours après la mise en oeuvre* | Essai réalisé 15 jours après la mise en oeuvre* | Essai réalisé 1 mois après la mise en oeuvre* | Essai réalisé 2 mois après la mise en oeuvre* |
|---|---|---|---|---|---|---|
| 2.1 | PP+GMS | Glissement faible | Glissement faible à modéré | Glissement modéré à important Présence de traces | Glissement important Présence de traces | Glissement important Beaucoup de traces |
| 2.2 | PP+1% du copolymère de l'exemple 2 | Glissement important | Glissement important | Glissement important | Glissement important | Glissement important |
| 2.3 | PP+ 2% du copolymère de l'exemple 2 | Glissement important | Glissement important | Glissement important | Glissement important | Glissement important |
| 2.4 | PP+ 5% du copolymère de l'exemple 2 | Glissement important | Glissement important | Glissement important | Glissement important | Glissement important |
| 2.5 | PP+ 10% du copolymère de l'exemple 2 | Glissement important | Glissement important | Glissement important | Glissement important | Glissement important |

| | | | | | | |
|---|---|---|---|---|---|---|
| **Chaque essai est réalisé 3 fois pour valider le résultat obtenu.* Glissement faible : pouvant entraîner une dégradation importante des implants (force appliquée >10N) Glissement modéré : nécessitant une force élevée pour l'injection avec le risque d'altération des implants (5N<force appliquée<10N) Glissement important : permettant une injection aisée sans risque d'altération des implants (1N<force appliquée<5N) | | | | | | |

Les résultats montrent que les embouts/cartouches élaborés en présence du copolymère de l'exemple 2 présentent les mêmes propriétés de glisse avec les implants hydrophiles que les implants hydrophobes.

### Exemple 10

Un polypropylène de référence commerciale PPR10222 de Total est "mélangé (« compoundé ») avec le copolymère de l'exemple 3 introduit à des pourcentages de 1, 2 et 5% dans les conditions suivantes :
- extrudeuse bi-vis Ø26mm L=50D
- Débit : 10kg/h
- Vitesse des vis : 300 rpm
- Profil de température : (pied) 210-210-200-190-180-170-170-160-160-160 (filière) : programmé ainsi en raison de la très grande fluidité du compound obtenu. La température est baissée en filière au voisinage de la température de fusion du PP. Chaque bloc de température fait 5D de long.

Les 2 composants polymères sont mélangés et introduits via un doseur gravimétrique en pied d'extrudeuse. Les différentes compositions métastables obtenues (« compounds ») sont récupérées en sortie d'extrudeuse sous forme de granulés et conservées à température ambiante.

Les différentes compositions métastables obtenues sont ensuite mises en oeuvre par injection moulage pour fabriquer des cartouches/embouts d'injecteurs d'implants ophtalmiques de diamètre de sortie 2 mm. Aucun problème de démoulage n'est constaté lors de la mise en oeuvre.

Les mesures de glisse ont été évaluées sur les injecteurs à intervalles réguliers sur 2 mois après leur date de fabrication. Tous les essais d'injection ont été réalisés avec des implants hydrophiles en HEMA (28% water content) de dioptrie 28D après ajout d'un visqueux d'acide hyaluronique. Deux cartouches/embouts de référence en PP ont été utilisées à titre de comparaison. La première contient 0,25% de GMS, la seconde, après avoir suivi un traitement par plasma, est revêtue d'un polymère hydrophile.

Les résultats sont rapportés dans le tableau 3 ci-après. Les cartouches/embouts fabriquées en une étape à partir de compositions métastables PP+copolymère de l'exemple 3 présentent des propriétés de glisse comparables aux cartouches/embouts traitées par plasma puis revêtues par un polymère hydrophile.

**Tableau 3**

| N° | Nature de la cartouche /embouts | Essai réalisé 1 jour après la mise en oeuvre* | Essai réalisé 7 jours après la mise en oeuvre* | Essai réalisé 15 jours après la mise en oeuvre* | Essai réalisé 1 mois après la mise en oeuvre* | Essai réalisé 2 mois après la mise en oeuvre* |
|---|---|---|---|---|---|---|
| 3.1 | PP+GMS | Glissement faible | Glissement faible - modéré | Glissement modéré - important | Glissement important Présence de traces | Glissement important Beaucoup de traces |
| 3.2 | PP+ revêtement hydrophile | Glissement important | Glissement important | Glissement important | Glissement important | Glissement important |
| 3.3 | PP+1% du copolymère de l'exemple 3 | Glissement important | Glissement important | Glissement important | Glissement important | Glissement important |
| 3.4 | PP+ 2% du copolymère de l'exemple 3 | Glissement important | Glissement important | Glissement important | Glissement important | Glissement important |
| 3.5 | PP+ 5% du copolymère de l'exemple 3 | Glissement important | Glissement important | Glissement important | Glissement important | Glissement important |

| | | | | | | |
|---|---|---|---|---|---|---|
| **Chaque essai est réalisé 3 fois pour valider le résultat obtenu.* Glissement faible : pouvant entraîner une dégradation importante des implants (force appliquée >10N) Glissement modéré : nécessitant une force élevée pour l'injection avec le risque d'altération des implants (5N<force appliquée<10N) Glissement important : permettant une injection aisée sans risque d'altération des implants (1 N<force appliquée<5N) | | | | | | |

En ce qui concerne les embouts/cartouches élaborés à partir des compositions métastables PP + copolymère de l'exemple 3, on peut constater une lubrification comparable à la voie revêtement ainsi que l'absence de toute trace sur les implants injectés. L'avantage principal de cette approche par rapport à la voie « revêtement » est la grande simplicité de la mise en oeuvre.

### Exemple 11

Un polyamide de type PEBAX 7033 d'Arkema est mélangé (« compoundé ») avec le copolymère de l'exemple 3.

La préparation des compositions métastables est réalisée selon le protocole de l'exemple 8 en utilisant une température d'injection de 260°C.

De même, les cartouches/embouts réalisés à partir de ces compositions métastables sont préparées selon le protocole de l'exemple 8 en utilisant une température de moule de 45-50°C. Les embouts préparés possèdent également un diamètre de sortie de 2 mm.

Concernant les propriétés de glisse, les essais d'injection d'implants ont été réalisés avec des implants hydrophiles en HEMA (28% water content) de dioptrie 27D après ajout d'un visqueux d'acide hyaluronique.

Deux cartouches/embouts de références, la première fabriquée à partir du même PEBAX chargé de 0,25% massique de GMS, la seconde constituée d'une cartouche/embout en PEBAX revêtue par un polymère hydrophile ont été utilisées pour permettre une comparaison avec les systèmes existants.

Les résultats sont rapportés dans le tableau 4 ci-après.

**Tableau 4**

| N° | Nature de la cartouche/em bout | Essai réalisé 1 jour après la mise en oeuvre* | Essai réalisé 7 jours après la mise en oeuvre* | Essai réalisé 15 jours après la mise en oeuvre* | Essai réalisé 1 mois après la mise en oeuvre* | Essai réalisé 2 mois après la mise en oeuvre* |
|---|---|---|---|---|---|---|
| 4.1 | PA**+GMS | Glissement faible | Glissement faible | Glissement modéré | Glissement important Présence de traces | Glissement important Présence de traces |
| 4.2 | PA+ revêtement hydrophile | Glissement important | Glissement important | Glissement important | Glissement important | Glissement important |
| 4.3 | PA+1% copolymère de l'exemple 3 | Glissement important | Glissement important | Glissement important | Glissement important | Glissement important |
| 4.4 | PA+ 2% copolymère de l'exemple 3 | Glissement important | Glissement important | Glissement important | Glissement important | Glissement important |
| 4.5 | PA+ 5% copolymère de l'exemple 3 | Glissement important | Glissement important | Glissement important | Glissement important | Glissement important |

| | | | | | | |
|---|---|---|---|---|---|---|
| **Chaque essai est réalisé* 3 *fois pour valider le résultat obtenu,**PA = polyamide* Glissement faible : pouvant entraîner une dégradation importante des implants (force appliquée >10N) Glissement modéré : nécessitant une force élevée pour l'injection avec le risque d'altération des implants (5N<force appliquée<10N) Glissement important : permettant une injection aisée sans risque d'altération des implants (1 N<force appliquée<5N) | | | | | | |

Les résultats montrent que les implants injectés par l'intermédiaire de cartouches/embouts fabriquées à partir de la composition métastable PP+ copolymère de l'exemple 3 glissent parfaitement et peuvent être aisément introduits à travers une incision de 2mm. Le glissant qui caractérise ces cartouches/embouts est en tout point comparable, voire meilleur, que celui des cartouches/embouts revêtues par un polymère hydrophile.

### Exemple 12

Les cartouches/embouts décrites dans l'exemple 11 ont servi à injecter des implants souples hydrophobes de dioptrie 26D ; le protocole utilisé est similaire à celui décrit pour les implants hydrophiles.

Le glissement est important pour toutes les cartouches/embouts fabriquées à partir de la composition métastable PA+copolymère de l'exemple 3. Les résultats sont superposables à ceux obtenus avec les implants hydrophiles.

### Exemple 13

Le copolymère polyuréthane de l'exemple 6 de masse molaire 25 500g/mol ayant deux extrémités acrylate a été compoundé avec le polypropylène PPR 10222 dans une extrudeuse à 240°C afin de préparer les compositions métastables correspondantes selon le protocole de l'exemple 8. Les compositions sont stockées à température ambiante.

La mise en oeuvre de la cartouche/embout est ensuite réalisée par injection/moulage des compositions métastables correspondantes en présence de péroxyde de dicumyle (un amorceur radicalaire) à 220°C. La présence de l'amorceur a pour objectif de permettre le greffage radicalaire d'une partie des chaînes de copolymère de fonction par leurs extrémités acrylate au polypropylène afin de créer des liens covalents dans la composition métastable. Ceci permet de disposer d'une surface hydrophile qui gonfle en présence d'eau mais ne se solubilise pas. Cette caractéristique en fait un dispositif particulièrement adapté aux systèmes utilisant des implants pré-chargés dans un liquide physiologique.

Pour valider le greffage chimique, les cartouches/embouts sont rincées 5 fois à l'eau et l'éthanol, les chaînes non fixées de façon covalente étant ainsi éliminées. L'analyse par microscopie Raman de la surface de la cartouche/embout montre la présence du copolymère après les différents lavages.

Le protocole retenu pour caractériser les propriétés de glisse est le suivant : l'implant hydrophile à base de polyhydroxyéthylméthacrylate (PHEMA) est placé dans la cartouche/embout puis le liquide physiologique est ajouté et le dispositif pré-chargé est conservé à température ambiante. Des essais d'injection d'implants sont ensuite réalisés à intervalles réguliers, à savoir après un jour, 7 jours, 15 jours, 30 jours et 60 jours d'immersion. Les résultats sont rassemblés dans le tableau 5 où ils peuvent être comparés à ceux obtenus avec une cartouche/embout commercialement disponible revêtue par un polymère hydrophile.

Les essais d'injection d'implants sont réalisés avec des implants de dioptrie 27D et le diamètre de sortie des cartouches/embouts est de 2mm.

Les résultats sont rassemblés dans le tableau 5 ci-après.

**Tableau 5**

| N° | Nature de la cartouche/embout | Essai réalisé après 1 jour d'immersion | Essai réalisé après 7 jours d' immersion | Essai réalisé après 15 jours d'immersion | Essai réalisé après 30 jours d'immersion | Essai réalisé après 60 jours d'immersion |
|---|---|---|---|---|---|---|
| 6.1 | PP + revêtement hydrophile | Glissement faible | Pas de glissement | Pas de glissement | Pas de glissement | Pas de glissement |
| 6.2 | PP + 1% copolymère acrylate de l'exemple 6 | Glissement modéré | Glissement modéré | Glissement modéré | Glissement modéré | Glissement modéré |
| 6.3 | PP + 2% copolymère acrylate de l'exemple 6 | Glissement modéré | Glissement important | Glissement important | Glissement important | Glissement important |
| 6.4 | PP + 5% copolymère acrylate de l'exemple 6 | Glissement modéré | Glissement important | Glissement important | Glissement important | Glissement important |

| | | | | | | |
|---|---|---|---|---|---|---|
| **Chaque essai est réalisé 3 fois pour valider le résultat obtenu.* Glissement faible : pouvant entraîner une dégradation importante des implants (force appliquée >10N) Glissement modéré : nécessitant une force élevée pour l'injection avec le risque d'altération des implants (5N<force appliquée<10N) Glissement important : permettant une injection aisée sans risque d'altération des implants (1N<force appliquée<5N) | | | | | | |

Les résultats montrent que le glissement est important pour des cartouches/embouts élaborés à partir d'une composition métastable contenant au moins 2% du copolymère présentant des groupements acrylate de l'exemple 6. Contrairement au système de référence à base de PP revêtu par un polymère hydrophile les dispositifs obtenus à partir des compositions métastables présentent un comportement identique après 2 mois avec toujours une glisse importante indiquant que le copolymère de fonction n'est pas lessivé, ni solubilisé par le liquide physiologique.

## Revendications

1. Composition polymère métastable comprenant un mélange d'au moins un polymère constitutif et au moins un copolymère de fonction partiellement miscibles ou compatibles, dans laquelle
ledit polymère constitutif est un polymère thermoplastique présentant une température de transition vitreuse (Tg) ou une température de fusion (Tf) supérieure ou égale à 80°C, et est présent dans le mélange en proportion massique comprise entre 85% et 99,9%, et
ledit copolymère de fonction a une température de transition vitreuse (Tg) ou une température de fusion (Tf) supérieure ou égale à 40°C et inférieure aux valeurs de Tg ou de Tf dudit polymère constitutif, possède au moins 60% massique d'unités monomères à caractère hydrophile, et des séquences ou blocs hydrophobes partiellement miscibles ou compatibles avec le polymère constitutif, et a une masse molaire supérieure à 5000g/mole.

2. Composition selon la revendication 1, **caractérisée en ce que** le polymère constitutif est choisi parmi les polyoléfines et plus particulièrement le polypropylène, les polyamides, les polyesters ou les polyuréthanes.

3. Composition selon l'une quelconque des revendications 1 ou 2, **caractérisée en ce que** le copolymère de fonction présente une partie hydrophile majoritaire, de préférence entre 60 et 99% massique d'unités monomères hydrophiles et en particulier entre 70 et 97% massique d'unités monomères hydrophiles.

4. Composition selon l'une quelconque des revendications 1 à 3, **caractérisée en ce que** le copolymère de fonction est choisi parmi les copolymères statistiques, les copolymères à blocs, les copolymères en peigne et les copolymères en étoile.

5. Composition selon l'une quelconque des revendications 1 à 4 **caractérisée en ce que** le copolymère de fonction est choisi parmi les polyéthers, les polyesters et les polyuréthanes présentant à la fois des séquences ou blocs hydrophiles et hydrophobes, et, les copolymères à base d'acide (méth)acrylique, d'oxyde d'éthylène, d'acrylamide, d'alcool vinylique, de vinyl pyrolidone ou d'hydroxyéthyl(méth)acrylate associés à des motifs hydrophobes partiellement miscibles ou compatibles avec le polymère constitutif.

6. Composition selon l'une quelconque des revendications 1 à 5, **caractérisée en ce que** le copolymère de fonction possède en outre des groupes réactifs aptes à réagir en présence de radicaux libres pour générer des liaisons covalentes entre les chaînes du copolymère de fonction et les chaînes du polymère constitutif.

7. Composition selon l'une quelconque des revendications 1 à 6, **caractérisée en ce qu'**elle se trouve dans un état morphologique figé, non évolutif, à des températures inférieures aux Tg/Tf des différents composants polymères du mélange et qu'elle est apte à évoluer vers un état morphologique multiphasique différent à des températures supérieures aux Tg/Tf des constituants polymères.

8. Utilisation d'une composition selon l'une quelconque des revendications 1 à 7 pour la fabrication d'un matériau, d'une pièce de dispositif médical ou d'un dispositif médical.

9. Utilisation selon la revendication 8, **caractérisée en ce que** le dispositif médical est un dispositif d'injection d'implant ophtalmique.

10. Matériau préparé à partir d'une composition métastable selon l'une quelconque des revendications 1 à 7.

11. Matériau selon la revendication 10, dans lequel le polymère constitutif de ladite composition métastable apporte les propriétés mécaniques au matériau préparé à partir de celle-ci et le copolymère de fonction apporte un caractère glissant audit matériau.

12. Pièce de dispositif médical élaborée à partir d'une composition métastable selon l'une quelconque des revendications 1 à 7, qui est tout ou partie d'un dispositif d'injection d'implant ophtalmique.

13. Dispositif d'injection d'implant ophtalmique comprenant au moins une pièce préparée à partir d'une composition métastable selon l'une quelconque des revendications 1 à 7.

14. Procédé de fabrication d'un matériau, d'une pièce de dispositif médical ou d'un dispositif médical, **caractérisé en ce qu'**il comprend une étape
d'injection/moulage d'une composition métastable selon l'une quelconque des revendications 1 à 7.

## Patentansprüche

1. Metastabile Polymerzusammensetzung mit einem Gemisch aus zumindest einem konstituierenden Polymer und zumindest einem funktionellen Copolymer, die teilweise mischbar oder verträglich sind, wobei das konstituierende Polymer ein thermoplastisches Polymer mit einer Glasübergangstemperatur (Tg) bzw. einer Schmelztemperatur (Tf) größer oder gleich 80 °C ist und in dem Gemisch in einem Massenanteil zwischen 85% und 99,9% vorliegt, und
das funktionelle Copolymer eine Glasübergangstemperatur (Tg) bzw. eine Schmelztemperatur (Tf) größer oder gleich 40 °C und niedriger als die Werte Tg bzw. Tf des konstituierenden Polymers aufweist, mindestens 60 Masse-% hydrophile Monomereinheiten und hydrophobe Sequenzen oder Blöcke besitzt, die teilweise mischbar oder verträglich mit dem konstituierenden Polymer sind, und eine Molmasse von mehr als 5000 g/mol hat.

2. Zusammensetzung nach Anspruch 1, **dadurch gekennzeichnet, dass** das konstituierende Polymer ausgewählt ist aus Polyolefinen und insbesondere aus Polypropylen, Polyamiden, Polyestern oder Polyurethanen.

3. Zusammensetzung nach einem der Ansprüche 1 oder 2, **dadurch gekennzeichnet, dass** das funktionelle Copolymer einen überwiegend hydrophilen Teil, vorzugsweise zwischen 60 und 99 Masse-% hydrophiler Monomereinheiten und insbesondere zwischen 70 und 97 Masse-% hydrophiler Monomereinheiten aufweist.

4. Zusammensetzung nach einem der Ansprüche 1 bis 3, **dadurch gekennzeichnet, dass** das funktionelle Copolymer ausgewählt ist aus statistischen Copolymeren, Blockcopolymeren, Kammcopolymeren und Sterncopolymeren.

5. Zusammensetzung nach einem der Ansprüche 1 bis 4, **dadurch gekennzeichnet, dass** das funktionelle Copolymer ausgewählt ist aus Polyethern, Polyestern und Polyurethanen mit sowohl hydrophilen als auch hydrophoben Sequenzen oder Blöcken, und Copolymeren auf Basis von (Meth)acrylsäure, Ethylenoxid, Acrylamid, Vinylalkohol, Vinylpyrrolidon oder Hydroxyethyl(meth)acrylat, assoziiert mit hydrophoben Einheiten, die teilweise mischbar oder verträglich mit dem konstituierenden Polymer sind.

6. Zusammensetzung nach einem der Ansprüche 1 bis 5, **dadurch gekennzeichnet, dass** das funktionelle Copolymer ferner reaktive Gruppen aufweist, die dazu geeignet sind, in Gegenwart von freien Radikalen zu reagieren, um kovalente Bindungen zwischen den Ketten des funktionellen Copolymers und den Ketten des konstituierenden Polymers zu bilden.

7. Zusammensetzung nach einem der Ansprüche 1 bis 6, **dadurch gekennzeichnet, dass** sie sich in einem starren, nicht-evolutiven morphologischen Zustand bei Temperaturen unterhalb von Tg/Tf der verschiedenen polymeren Komponenten des Gemischs befindet und dass sie geeignet ist, bei Temperaturen oberhalb von Tg/Tf der konstituierenden Polymere in einen anderen mehrphasigen, morphologischen Zustand überzugehen.

8. Verwendung einer Zusammensetzung nach einem der Ansprüche 1 bis 7 zum Herstellen eines Materials, eines Teils eines medizinischen Geräts oder eines medizinischen Geräts.

9. Verwendung nach Anspruch 8, **dadurch gekennzeichnet, dass** das medizinische Gerät ein Augenimplantat-Injektionsgerät ist.

10. Material, hergestellt aus einer metastabilen Zusammensetzung nach einem der Ansprüche 1 bis 7.

11. Material nach Anspruch 10, wobei das konstituierende Polymer der metastabilen Zusammensetzung dem daraus hergestellten Material die mechanischen Eigenschaften verleiht und das funktionelle Copolymer dem Material einen gleitenden Charakter verleiht.

12. Teil eines medizinischen Geräts, hergestellt aus einer metastabilen Zusammensetzung nach einem der Ansprüche 1 bis 7, bei dem es sich ganz oder teilweise um ein Augenimplantat-Injektionsgerät handelt.

13. Augenimplantat-Injektionsgerät mit zumindest einem Teil, hergestellt aus einer metastabilen Zusammensetzung nach einem der Ansprüche 1 bis 7.

14. Verfahren zum Herstellen eines Materials, eines Teils von einem medizinischen Gerät oder eines medizinischen Geräts, **dadurch gekennzeichnet, dass** es einen Schritt des Spritzens/Formens einer metastabilen Zusammensetzung nach einem der Ansprüche 1 bis 7 umfasst.

## Claims

1. Metastable polymer composition comprising a mixture of at least one constituent polymer and at least one partially miscible or compatible functional copolymer, in which
said constituent polymer is a thermoplastic polymer having a glass transition temperature (Tg) or a melting temperature (Tm) above or equal to 80°C, and is present in the mixture in a mass proportion comprised between 85% and 99.9%, and
said functional copolymer has a glass transition temperature (Tg) or a melting temperature (Tm) above or equal to 40°C and below the Tg or Tm values of said constituent polymer, comprises at least 60% by mass of monomer units with a hydrophilic character, and hydrophobic sequences or blocks partially miscible or compatible with the constituent polymer, and a molar mass above 5000g/mol.

2. Composition according to claim 1, **characterized in that** the constituent polymer is chosen from the polyolefins and more particularly polypropylene, polyamides, polyesters or polyurethanes.

3. Composition according to any one of claims 1 or 2, **characterized in that** the functional copolymer comprises a majority hydrophilic part, preferably between 60 and 99% by mass of hydrophilic monomer units and in particular between 70 and 97% by mass of hydrophilic monomer units.

4. Composition according to any one of claims 1 to 3, **characterized in that** the functional copolymer is chosen from random copolymers, block copolymers, comb copolymers and star copolymers.

5. Composition according to any one of claims 1 to 4, **characterized in that** the functional copolymer is chosen from the polyethers, polyesters and polyurethanes having both hydrophilic and hydrophobic sequences or blocks, and copolymers based on (meth)acrylic acid, ethylene oxide, acrylamide, vinyl alcohol, vinyl pyrrolidone or hydroxyethyl(meth)acrylate combined with hydrophobic units partially miscible or compatible with the constituent polymer.

6. Composition according to any one of claims 1 to 5, **characterized in that** the functional copolymer moreover comprises reactive groups capable of reacting in the presence of free radicals in order to generate covalent bonds between the chains of the functional copolymer and the chains of the constituent polymer.

7. Composition according to any one of claims 1 to 6, **characterized in that** it is found in a non-changing set morphological state, at temperatures below the Tg/Tm of the different polymer components of the mixture and **in that** it is capable of changing towards a different multiphase morphological state at temperatures above the Tg/Tm of the polymer constituents.

8. Use of a composition according to any one of claims 1 to 7 for manufacturing a material, a medical device part or a medical device.

9. Use according to claim 8, **characterized in that** the medical device is an ophthalmic implant injection device.

10. Material prepared from a metastable composition according to any one of claims 1 to 7.

11. Material according to claim 10, in which the constituent polymer of said metastable composition provides the material prepared from it with mechanical properties and the functional copolymer provides said material with a character of slipperiness.

12. Medical device part prepared from a metastable composition according to any one of claims 1 to 7, which forms all or part of an ophthalmic implant injection device.

13. Ophthalmic implant injection device comprising at least one part prepared from a metastable composition according to any one of claims 1 to 7.

14. Method for manufacturing a material, a medical device part or a medical device, **characterized in that** it comprises a step of injection/moulding of a metastable composition according to any one of claims 1 to 7.
